# EUROPEAN PATENT SPECIFICATION

(11) **EP 2 020 973 B1**
(45) Date of publication and mention of the grant of the patent: **13.11.2019**
(21) Application number: 06755918.7
(22) Date of filing: 26.05.2006
(51) Int. Cl.: A61F 13/496, A61F 13/515, A61F 13/15

(54) **HYGIENE PANTS FOR SINGLE USE**
HYGIENE-HÖSCHEN ZUM EINMALIGEN GEBRAUCH
CULOTTE HYGIÉNIQUE À USAGE UNIQUE

(43) Date of publication of application: 11.02.2009
(60) Divisional application: 09157176.0
(73) Proprietor: Essity Hygiene and Health Aktiebolag, 405 03 Göteborg (SE)
(72) Inventor: CARTIER, Jean-Francois, Québec J2B 1M4 (CA); DUMAS, Christian, Québec J2B 6V1 (CA); EEN, Hans, 435 41 Mölnlycke (SE); LEHTO, Marcus, 430 92 Fotö (SE)
(74) Representative: Valea AB
(86) International application number: PCT/IB2006/001397
(87) International publication number: WO 2007/138373

(56) References cited:
- EP-A1- 1 491 105
- EP-A2- 0 875 226
- EP-A2- 0 990 434
- WO-A-2006/093444
- WO-A1-2007/008128
- FR-A- 2 873 545
- US-A- 5 769 838
- US-A1- 2004 102 757
- US-B1- 6 716 778

## Description

### TECHNICAL FIELD

The present invention relates to disposable hygiene pants, such as diaper pants.

### BACKGROUND

Very high demands are placed on fit and comfort for hygiene pants such as diaper pants for children and sanitary towel pants for adults. The hygiene pants preferably have sufficient elasticity in the transverse direction (*i.e*., the horizontal direction when the pants are held upright) that they are easy to take off and put on by the user, a parent or a care provider. There is also a preference that they should be soft and textile-like. Hygiene pants, in particular for adult users, are preferably discrete and not bulky, at least in the areas outside the actual absorption unit. Since they are disposable products, the cost is preferably minimized, in particular the costs of materials and manufacture. For cost reasons, the material layers included are preferably as thin as possible. At the same time, quality and strength requirements must be fulfilled.

Hygiene pants, such as diaper pants, preferably resist wear and breakage during use. Hygiene pants can be placed under significant stresses when being put on, for example the pants are stretched when they are being pulled over the user's hips. In particular, longitudinal weld seams connecting front portions to back portions of the garment at the sides of the pant are exposed to considerable stresses.

In traditional hygiene pants, covers have been made of double nonwoven layers with elastic threads lying between these. In welded side seams on this type of cover, there are no fewer than four layers of bonded nonwoven, in which the bonds have not been broken open or weakened in connection with the production of the cover, which can provide a sufficiently strong weld seam.

WO 03/047488 discloses an elastic laminate composed of an elastic film applied between two nonwoven layers. During production of the elastic laminate, these nonwoven layers have been connected to the film, after which the laminate has been stretched until bonds in the nonwoven layer have been broken. As a result, the elasticity of the laminate is largely the same as the elasticity of the elastic film. One disadvantage of this solution is that weld seams in hygiene pants constructed with covers made of such laminates can have considerably less strength than the weld seams on traditional diaper pants.

Other examples of elastic laminates used in construction of hygiene pants are described in PCT applications WO 2005/122984 and WO 2005/122985. In contrast to the production method disclosed in WO 03/047488, the bonds of at least one nonwoven layer have not been completely broken, instead, the layer retains a certain residual strength. PCT applications WO 2005/122984 and WO 2005/122985 describe laminates in which elasticity has been combined with softness and resistance to puncture. However, welded side seams of hygiene pants constructed of laminates described in PCT applications WO 2005/122984 and WO 2005/122985 can still be weaker that the weld seams of traditional pants.

EP 0 990 434 A2 discloses a disposable pull-on-diaper with seams joined by a plurality of heat-sealing zones.

Other disposable absorbent articles are described in EP0875226, and under A54(3)EPC in WO2006/093444 and WO2007/008128.

As can be seen from the above, many demands, some of them contradictory, are placed on disposable hygiene pants. Improvements in the art are still to be desired.

### SUMMARY

Described herein are disposable hygiene pants, such as diaper pants. The pants can be described with reference to a longitudinal direction, which corresponds to the vertical when the pants are held upright, and a transverse direction, which is a horizontal direction from side to side as the pants are held upright. The pants comprise in the longitudinal direction, a front portion, an intermediate crotch portion provided with leg opening cutouts, and a rear portion, the outer longitudinal edge portions of the front portion being connected by longitudinal weld seams to the outer longitudinal edge portions of the rear portion in order to form a waist opening delimited by the outer transverse edge portions of the front portion and of the rear portion and forming two leg openings in the crotch portion, said leg openings being delimited in the transverse direction by inner end portions of the weld seams. The hygiene pants comprise an absorption unit extending in the longitudinal direction across at least part of the crotch portion. The hygiene pants also comprise an outer cover that is made of elasticized material comprising an elastic film applied to a layer of nonwoven material or a layer of elastic film applied between two layers of nonwoven material.

The hygiene pants are characterized in comprising weld reinforcing pieces of material that comprise thermoplastic fibers, such as bonded nonwoven material, adhesively affixed proximate to each weld seam on at least one of said front portion and/or said back portion, such that a portion of a weld reinforcing piece of material extends into each of said weld seams between said longitudinal edge portions of said front portion and said back portion. Weld reinforcing pieces of material are adhesively affixed to the inner surface of the front and/or back portions of the elastic outer cover, and most preferably the adhesive is positioned proximate to the weld seam such that it does not extend into the area of the weld seam. As used herein, proximate to the weld seam means close to the weld seam, for example at least within about 5 cm, preferably at least within about 3 cm and most preferably at least in a location within about 1 cm of the weld. Generally, it is preferred to place the adhesive inboard of the weld seam and as close as practical to the location of the weld seam without extending into the weld seam, but this is not to exclude the reinforcing material also being affixed by adhesive that is less proximate to the weld seam, for example in the case of a piece of reinforcing material that extends transversely across either the front or back portion adhesive may be used continuously or at discrete points across the width of the reinforcing material. Additional pieces of reinforcing material may be applied so as to extend into each weld with or without being adhesively affixed.

Accordingly, in preferred embodiments the hygiene pants may be characterized in that the weld seams are each reinforced by at least one reinforcing strip containing thermoplastic fibers, which reinforcing strip has been affixed to the front portion or rear portion of the hygiene pants at least along said inner end portions, as seen in the longitudinal direction of the hygiene pants, of the two edge portions and has been welded together there with said longitudinal edge portions of the cover in order to form said weld seams. Reinforcing strips are preferably composed of nonwoven spunbond or meltblown material or the like. A reinforcing strip is at least sufficiently wide in the transverse direction to accommodate being adhesively affixed near the weld, but not in the area of the weld. The reinforcing strip may span the transverse width of the pant on the front and or back portions, such a strip can be adhesively affixed to either the front or back portion using adhesive at a location proximate to the weld and the strip may also be adhesively affixed at points that are less proximate to the weld seam.

The hygiene pants are also characterized in that the tensile strength of the weld seams, in a direction transverse to the weld seam, exceeds 5 N/25.4 mm, more preferably the minimum tensile strength exceeds 10 N/25.4 mm or 12 N/25.4 mm and can be on average at least 15 N/25.4 mm or 20 N/25.4 mm in the portions reinforced with said nonwoven strip, where the breaking strength of the cover in the transverse direction preferably exceeds the tensile strength for the reinforced weld seams.

According to one embodiment, a hygiene pant may be characterized in that the longitudinal edge portions of both the front portion and the rear portion of the cover, at least along end portions thereof in the vicinity of the waist opening of the pant and/or in the vicinity of the leg opening, have been provided with reinforcing strips. In one embodiment, the hygiene pants may be characterized in that a reinforcing strip is adhesively affixed to the cover proximate to the weld seams in each of said longitudinal edge portions of either the front portion or the rear portion, and in that a reinforcing strip is applied with or without adhesive to each longitudinal edge portion of the other of the front portion or the rear portion, as a result of which the weld seams, at least in their reinforced areas, comprise two layers of said reinforcing nonwoven strips.

In some embodiments, the pant may be characterized in that when the weld seam is made, either the front or the back portion is disposed toward an ultrasonic horn and the other of the front or back portion is disposed toward an anvil and wherein the reinforcing strips of material are applied only to the inner surface of the front or back portion, preferably on the inner surface of the portion that is contacted by the ultrasonic horn. In some embodiments in which this arrangement is preferred, when the weld seam is being made, the ultrasonic horn is stationary with respect to the transverse direction of the pant and the anvil is moving in the transverse direction of the pant. Alternatively, where the reinforced weld is weaker between the front portion and the reinforcing strip or between the back portion and the reinforcing strip, the reinforcing strip is preferably adhesively affixed proximate to the weld seam on whichever of the front or back portion is on the weaker side.

In some embodiments, the pant may be characterized in that a reinforcing strip is composed of a single strip extending transversely across the hygiene pants. In some embodiments, the pant may be characterized in that, in the longitudinal direction of the hygiene pants, a strip extends only across said inner end portions of the two transverse edge portions.

A method of forming a weld seam which does not form part of the invention is also described. The weld seam is formed between a first material, and a second material, preferably elastic materials such as an elastic laminate. The method comprises adhesively adhering a reinforcing material comprising thermoplastic fibers to at least one of said first and second materials proximate to the location of the weld such that a potion of said reinforcing material extends into the location of the weld seam between said first and second materials. The adhesive can be placed such that it is proximate to, but not in the area of the weld seam. Where the weld seam is formed along a longitudinal direction between materials that are moving in a transverse direction by using a stationary ultrasonic horn and an anvil moving or rotating in the transverse direction at a rate equivalent to the first and second materials such that said first material is disposed toward the horn and the second material is disposed toward the anvil as the weld is being formed, the reinforcing material is adhesively adhered to at least the first material. Alternatively, where the reinforced weld seam is weaker between the first material and the reinforcing material, or between the second material and the reinforcing material, the reinforcing material is preferably adhesively affixed to whichever of the first or second materials is on the weaker side.

### DESCRIPTION OF THE FIGURES

Figure 1 shows a schematic perspective view of an illustrative embodiment of hygiene pants as described herein.
Figure 2 shows a cross section along line II-II in Figure 1.
Figure 3 shows a schematic perspective view of another illustrative embodiment of hygiene pants as described herein.
Figure 4 shows a cross section along line IV-IV in Figure 3.
Figure 5 shows a schematic perspective view of another illustrative embodiment of hygiene pants as described herein.
Figure 6 shows a cross section along line VI-VI in Figure 5.
Figure 7 shows a schematic perspective view of another illustrative embodiment of hygiene pants as described herein.
Figure 8 shows a cross section along line VIII-VIII in Figure 7.
Figures 9-11 show, schematically, a method for measuring the weld strength of the hygiene pants according to an embodiment of the present invention.
Figure 12 shows a strain/load diagram to illustrate the change in weld strength with a reinforcement applied without adhesive as described herein.
Figures 13-15 illustrate a method of making a reinforced weld seam between layers of elastic laminate which does not form part of the present invention.

### DETAILED DESCRIPTION OF PREFERRED EMBODIMENTS

Elastic laminates have various desirable attributes when used as elastic covers for hygiene pants. A laminate comprising an elastic film between two nonwoven layers can give the cover desired elastic properties while the fibers in these nonwoven layers give the cover a soft textile feel. The structure of the laminate is preferably configured such that elastic stretching of the cover is not impeded by the nonwoven layers. However, the strength of weld seams of hygiene pants having elastic laminate covers can be adversely affected by the structure and make-up of the laminate, in which the fiber bonds have been partially or completely broken. In some elastic laminate cover materials, the fibers of the nonwoven layers have been more or less stretched to the breaking point in the manufacture of the laminate.

It has also been discovered that typical methods of constructing weld seams such as used in hygiene pants can affect the strength of the weld seams in surprisingly adverse ways. In particular it has been discovered that in a high-speed production line using a stationary ultrasonic horn and a transversely moving anvil to form a weld along a longitudinal direction, the weld may be significantly weaker, and that when the weld involves more than two layers the weld between layers on the horn side tend to be weaker. Without wishing to be bound by theory, it is believed that this phenomenon can be explained by the breakage of fibers in the transversely moving material while in contact with the stationary horn.

By means of the methods described herein, the risk of welded side seams in hygiene pants prematurely breaking can be significantly reduced, so that elastic laminate materials can be safely and economically used to construct improved hygiene pants.

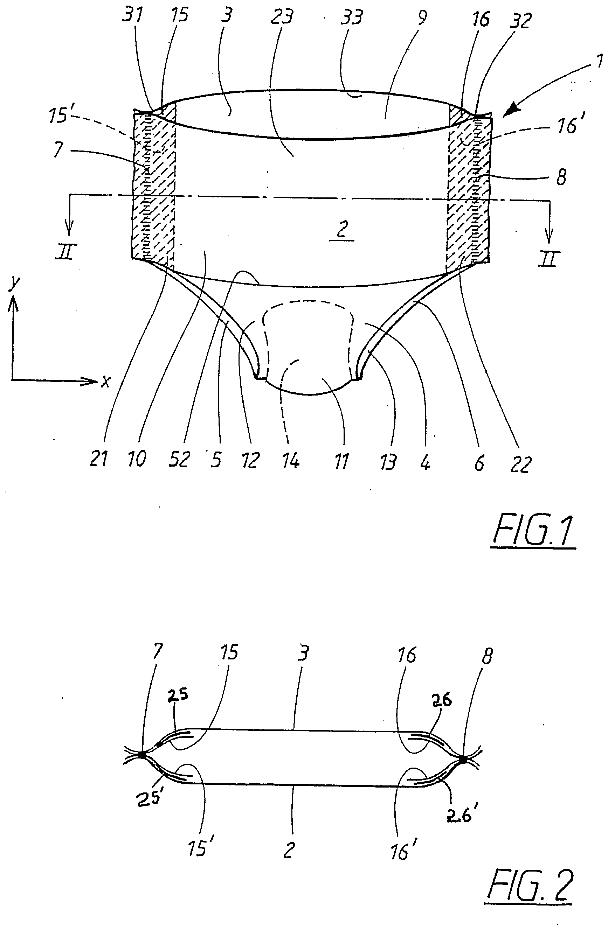

Figure 1 shows hygiene pants in the form of diaper pants 1 for children or incontinent adults. The diaper pants 1 have a front portion 2, a rear portion 3 and a crotch portion 4 at which leg openings 5, 6 are arranged. The diaper pants have a transverse direction, which in Figure 1 has been marked with x, and a longitudinal direction, which has been marked with y. The outer longitudinal edge portions 21 and 22 of the front portion, as seen in the transverse direction, are connected by weld seams 7 and 8 to the outer longitudinal edge portions 31 and 32 of the rear portion 3, as seen in the transverse direction, in order to form a waist opening 9, which is delimited by the outer transverse edge portions 23 and 33 of the front portion and rear portion.

The hygiene pants include an outer cover 10. The cover 10 is elastic, and comprises an elastic laminate containing an elastic film applied between two nonwoven layers. Three-layer elastic laminates suitable for the purpose are described in detail, for example, in PCT applications WO 2005/122984 and WO 2005/122985. A suitable two layer elastic laminate is described in PCT Patent Applications PCT/SE2006/000563 and PCT/SE2006/000564.

The laminate is elastic at least in the transverse direction of the diaper pants, that is, in the x direction in Figure 1. The elasticity in the x direction is preferably at least 30%, more preferably at least 50%, and most preferably at least 70%, measured according to the elasticity test described in the abovementioned patent applications.

The outer fiber layers in the laminate can provide softness and a textile feel. Examples of suitable materials are metlblown nonwovens and spunbond nonwovens. The grammage of said fibrous layers is preferably between 10 and 35 g/m², preferably between 12 and 30 g/m², and particularly preferably between 15 and 25 g/m². Examples of suitable fiber materials such as polyethylene and polypropylene are indicated in the abovementioned patent applications. The elastic film is expediently perforated so that the cover is made permeable to air and vapour. The grammage of the elastic film is expediently between 10 and 120 g/m², preferably between 20 and 60 g/m². Suitable examples of materials and material combinations for the elastic film are indicated in abovementioned patent applications WO 2005/122984 and WO 2005/122985.

Laminate in the cover 10 can be produced according to a modified version of the method disclosed in WO 03/047488. As has been described in said patent applications WO 2005/122984 and WO 2005/122985, the modification is that at least one of the non-elastic nonwoven layers has been stretched under maximum load so that significant strength is still present in at least one of the nonwoven layers.

In other embodiments, materials such as those described in PCT Patent Applications PCT/SE2006/000563 and PCT/SE2006/000564 can be used in constructing the cover. Elastic panels as described therein are formed by preparing a two-layer laminate comprising a non-elastic fibrous nonwoven web and an elastic film. Suitable materials for such laminates are described therein. The two layer laminate is activated by stretching in at least one direction by 10 200% to render the laminate elastic in at least that direction. In a pant-forming process, stretched laminate can be laminated to another chassis component web.

Alternatively, the laminate for the cover can also be composed of an elastic laminate which has been produced entirely in accordance with what is described in WO 03/047488
The term "cover" used herein designates a framework that supports the pants and that preferably elastically surrounds a user's trunk, thereby supporting the pants as a whole. In the illustrative embodiment shown in Figure 1, the cover is composed of a front portion 2 and a rear portion 3. The diaper pants according to Figure 1 have a crotch portion 4 welded to the cover and in the form of an absorption unit 11. This absorption unit 11 has an outer liquid-tight sheet 12, an inner liquid-permeable sheet 13, and an absorption body 14 arranged between these sheets. The liquid-permeable sheet can, for example, be composed of a nonwoven, such as a spunbond. The outer liquid-tight sheet 12 can, for example, be composed of a thin plastic film, such as a film of polyethylene or polypropylene. The absorption body can, for example, be composed of fluff pulp of cellulose or absorbent foam, possibly in combination with superabsorbent material. Other suitable materials and material combinations for the liquid-tight sheet 12, the absorption body 14 and the liquid-permeable sheet 13 are set out in patent applications WO 2005/122984 and WO 2005/122985.

Hygiene pants as described in WO 03/047488 and the hygiene pants as described in patent publications WO 2005/122984 and WO 2005/122985 and PCT Patent Application PCT/SE2006/000563 comprise elastic laminates with a degree of elasticity suitable for use in hygiene pants. This has been achieved by partially or completely breaking fibers or bonds in at least one of the nonwoven layers surrounding the elastic films in the elastic laminate. An important disadvantage of the nonwoven bonds in the laminate having been partially or completely broken is hat the tensile strength of weld seams 7 and 8 is weakened by comparison to traditional diaper pants that comprise four bonded nonwoven layers connected by means of the weld seams.

Bonded nonwoven layers here designate nonwovens which have not been broken open or weakened during production of the cover, but which instead retain their tensile strength. In traditional diaper pants, the covers are composed of double nonwovens and of elastic threads lying between them. In the weld seams, therefore, there are no less than four layers of bonded nonwovens, thus providing a sufficiently strong weld seam.

Hygiene pants are subjected to considerable stresses when being put on. The hygiene pants are stretched and the weld seams are exposed to considerable stresses. Especially critical areas are the ends of the weld seams at the leg openings or the waist.

In the embodiment according to Figure 1, the weld seams 7 and 8 have been reinforced with reinforcing material strips 15, 15' and 16, 16', which strips have been applied to the inside of the cover along the edge portions 21, 31 and 22, 32, as can be seen in Figure 2. Preferably, reinforcing strips 15 and 16 are adhesively affixed to rear portion 3 at adhesive locations 25, 26 proximate to welds 7,8 but not in the areas of welds 7,8 and reinforcing strips 15' , 16' are adhesively affixed to front portion 2 at adhesive locations 25', 26' proximate to welds 7,8 but not in the areas of welds 7,8.

The reinforcing strips contain thermoplastic fibers, which, upon welding of the weld seams, reinforce the seam. Nonwoven materials are preferred. For example, meltblown materials and spunbond materials are suitable reinforcing materials. However, other flexible and optionally extensible materials may be used, for example Spunbond-Meltblown-Spunbond (SMS) laminates.

In alternative embodiments, it can be sufficient to include only pieces 15,16 or pieces 15',16' on either the back or front portions of the pants. Where the welds 7,8 are formed in diaper pants moving in a transverse direction and using a stationary ultrasonic horn and a transversely moving anvil, it is preferable to include at least pieces 5,16 or 15',16' affixed to whichever of the front or back portions are disposed toward the stationary horn as the weld is formed.

The reinforcing strips 15,16 can, for example, be made of spunbond and applied to the edge portions of the cover before these are welded together. As stated, the strips are preferably adhesively attached to the cover at the time the strips are applied. The reinforcing strips are preferably not stretched on application to the edge portions of the cover, thus the fiber bonds have not been weakened. When the edge portions are welded together, the reinforcing strips can retain substantially all of their tensile strength, in contrast to fiber layers of elastic material in which the fiber bonds have been partially or completely broken.

Thus, the presence of the reinforcing strips in the welds can provide reinforcement of the bond between the cover layers. In addition, by adhesively affixing the reinforcing strip to an inner surface of the cover material at a region proximate to the weld, the reinforcing strip can provide a redundant connection. If the welded bond formed between an elastic cover portion and a reinforcing strip fails as a result of weakened or broken fiber bonds in the elastic cover material, the adhesive attaching the reinforcing strip to the elastic cover can maintain the integrity of the seam.

This is because, the welded bond between reinforcing strips and/or between a reinforcing strip and a cover portion that is disposed towards an anvil that is moving transversely with the hygiene pant as a when a longitudinal ultrasonic weld seam is formed at the transverse edges of transversely moving hygiene pants tends to be stronger than the bond between two directly welded elastic cover sheets or an elastic cover sheet and a reinforcing strip that is disposed towards a stationary horn when a longitudinal ultrasonic weld seam is formed at the transverse edges of transversely moving hygiene pants.

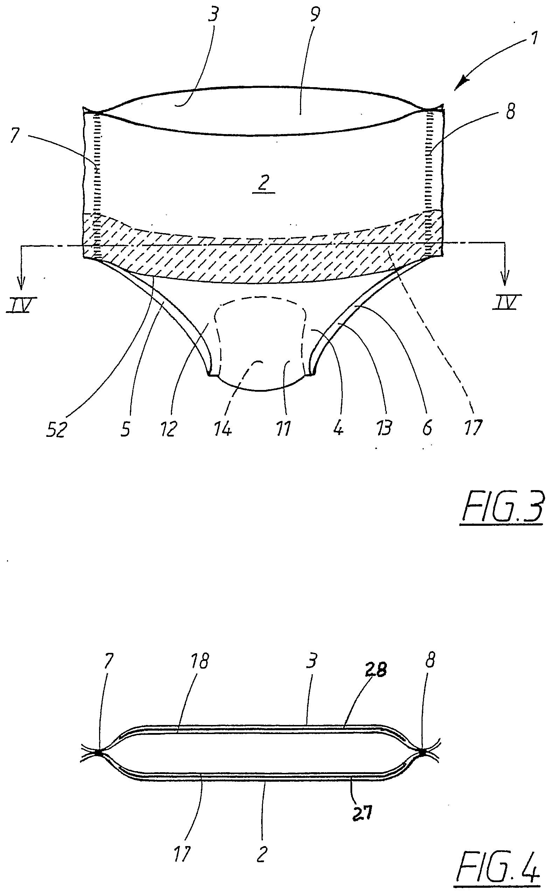

As has been mentioned above, the stresses on the weld seams are considerable at the leg openings. In the embodiment shown in Figures 3 and 4, two reinforcing strips 17, 18 have been applied to the inside of the cover across the hygiene pants and only across the inner end portions of the end portions of the cover, i.e. at the leg openings. The reinforcing strips 17, 18 are applied, one on the front portion and one on the rear portion, before these portions have been welded together by means of the weld seams to form the cover. Preferably, strips 17, 18 are adhesively affixed to back and front portions 3, 2 by adhesive 27, 28, which is applied at least proximate to the areas of welds 7, 8, but not in the area where the weld is formed. In Figures 3 and 4, the details corresponding to equivalent details in the illustrative embodiment in Figure 1 have been provided with the same reference numbers.

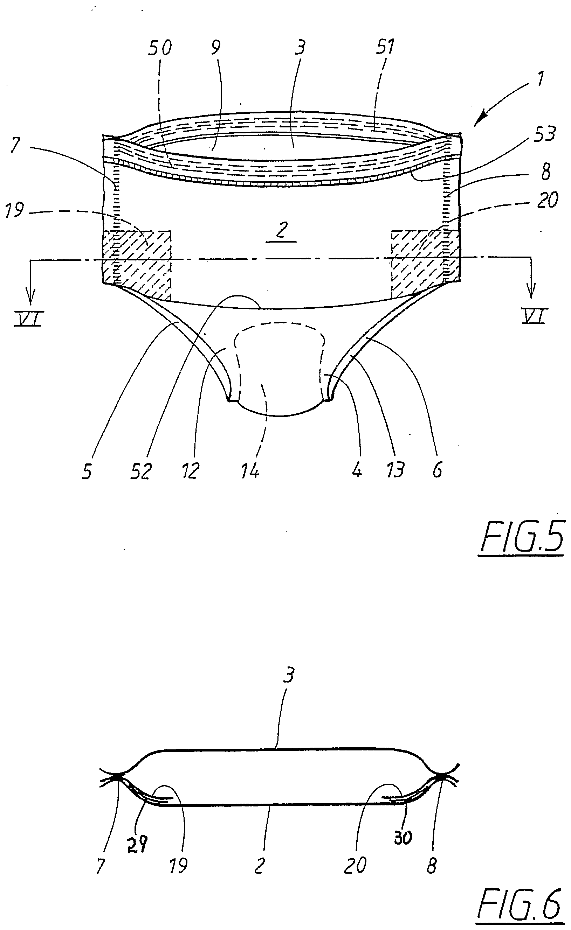

Figures 5 and 6 show diaper pants where only the edge portions of the cover in immediate proximity to the leg openings have been reinforced with strips 19 and 20. In this embodiment, only one strip has been applied inside the front portion 2 of the cover at the leg opening. In an alternative not shown, strips 19, 20 could be applied to back portion 3. Where the weld seams 7,8 are formed in transversely moving pants by using a stationary ultrasonic horn and a transversely moving anvil, it is preferable that strips 19, 20 are adhesively affixed to whichever of the front or back portion of the pant is disposed toward the stationary horn. In Figures 5 and 6, the details corresponding to equivalent details in the embodiment according to Figures 1 and 2 have been provided with the same reference numbers.

In the embodiment according to Figure 5, the front portion and rear portion of the hygiene pants have been provided with elastic waist bands 50 and 51. These waist bands can expediently be composed of two nonwoven layers with a number of elastic threads applied between the nonwoven layers. The waist bands are connected to the front portion and the rear portion by means of transverse weld joins of which one 53 has been indicated in Figure 5. When welding together the front portion and rear portion, the waist bands 50 and 51 are welded together by means of the weld seams 7 and 8. By means of the arrangement of the waist bands, the weld seams 7 and 8 are reinforced in the critical portions nearest to the waist opening. In preferred embodiments, the attachment of the nonwoven layers of the waist bands to the front and back portions of the cover using glue applied longitudinally and proximate to the welds 7,8.

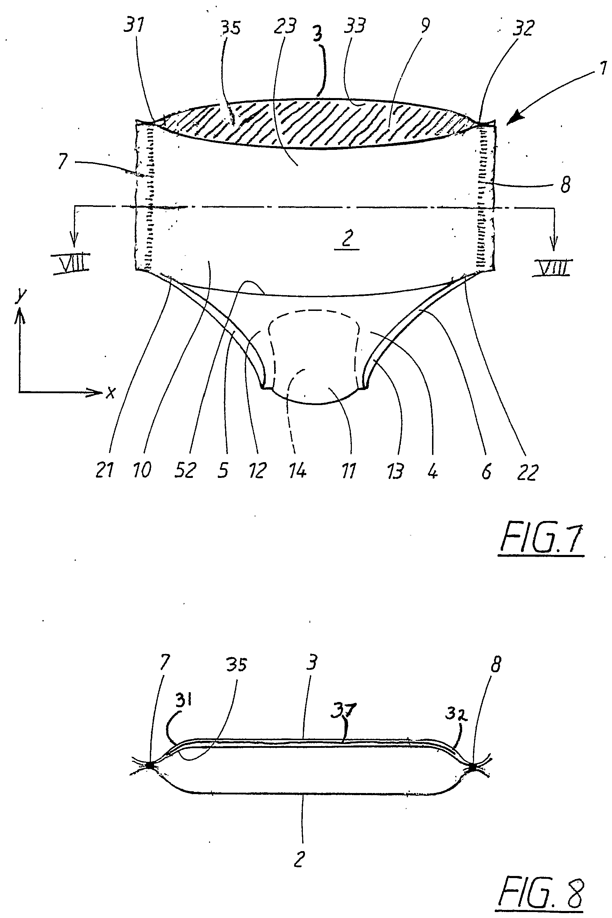

Figures 7 and 8 show diaper pants in which reinforcing nonwoven material 35 is applied across the width of the back portion. The reinforcing material 35 preferably extends in the longitudinal direction for substantially the length of welds 7,8. A reinforcing web, such as a bonded nonwoven web, laminated to the elastic panel may serve as both a cover layer and a reinforcing material, which is preferred when the cover is constructed using a two-layer elastic laminate such as described in PCT Patent Applications PCT/SE2006/000563 and PCT/SE2006/000564. The reinforcing material is preferably adhesively affixed to an inside surface of the elastic laminate cover while the cover is in a transversely extended state, for example the elastic laminate panel may be extended in the transverse direction about 10% to 200%, preferably about 20% to about 125%, more preferably about 50% to about 70% at the time the laminate and reinforcing material are affixed.

In the illustrated embodiment, only one piece of reinforcing material has been applied inside the back portion 3 of the cover. In an alternative not shown, a single piece of reinforcing material could be applied to front portion 2. Reinforcing strips that do not extend transversely across the width of the pant may be combined with reinforcing material that does extend across the width of the pant.

Where the weld seams 7,8 are formed in transversely moving pants by using a stationary ultrasonic horn and a transversely moving or rotating anvil, it is preferable that reinforcing material 35 is adhesively affixed to whichever of the front or back portion of the pant is disposed toward the stationary horn. Alternatively, pieces of reinforcing material may be applied to both the front and back portions. In Figures 7 and 8, the details corresponding to equivalent details in the embodiment according to Figures 1 and 2 have been provided with the same reference numbers.

In the illustrative embodiments described above, the cover comprises front and rear portions and a separate crotch portion that has been welded to the rest of the cover by means of a weld which has been indicated by 52 in the drawings. Thus, in various exemplary embodiments, the cover may be made of one piece of elastic laminate or of separate pieces of material with some pieces being substantially non-elastic. The diaper pants according to Figures 1-8 are illustrated with a crotch portion which is substantially non-elastic under the application stresses.

Alternatively, the cover can also comprise the crotch portion, i.e. so that front portion, rear portion and crotch portion are in one piece of elastic material. The absorption unit is applied in this case in the crotch portion inside the cover and connected to the latter, for example by means of melt adhesive.

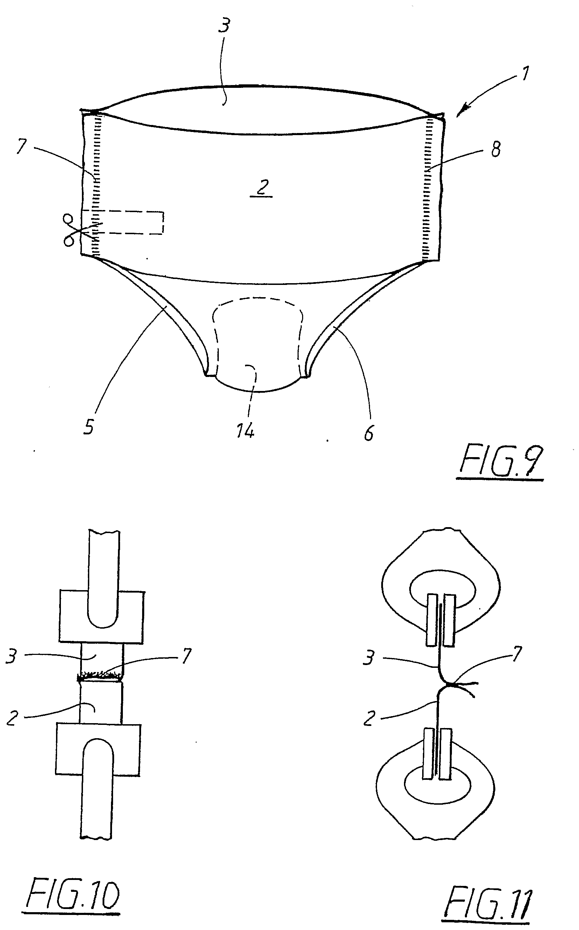

The tensile strength of the weld seams is measured according to the method (reference ASTM D 882) described in patent application PCT WO 2005/122984. Test specimens are cut from the products, as shown in Figure 9, in areas which are reinforced with nonwoven strips. The width of the test specimens will be 25.4 mm and the length if possible 50 mm longer than the distance between the clamps on the tensile test instrument Instron 4301. Figures 10 and 11 show how the test specimens are secured in the clamps.

To illustrate the effect of the reinforcement of the weld seams by means of nonwoven strips, Tables 1 and 2 show test results for measurements of the tensile strength of a weld seam for connecting two layers of an elastic laminate with and without reinforcement by nonwoven strips in various configurations. Table 1 provides a comparison of results obtained without reinforcing strips versus using reinforcing strips that are not adhesively affixed to the elastic laminate cover. Table 2 shows results that can be obtained using reinforcing strips with and without adhesively affixing one reinforcing strip to the elastic laminate cover at a location proximate to each weld, but not in the welded area. Table 2 further provides comparisons of higher basis weight reinforcing strip materials and the combination of a strip that is not adhesively affixed with a strip that is adhesively affixed.

The elastic laminate used in the test specimens comprises an inner elastomeric, three-ply film of PE-SEBS-PE provided with holes and having a grammage of 36 g/m² and two outer sheets of spunbond nonwoven, polypropylene, which each have a grammage of 22 g/m². The laminate has been produced according to a modified version of the method described in WO 03/04788. According to the modified version, a spunbond sheet is applied to the film in a tacky state and thus binds to the film, while the other spunbond sheet is adhesively laminated to the film by use of, for example, a pressure-sensitive adhesive (adhesive quantity 3 g/m²). The laminate is stretched gradually until the non-elastic spunbond sheets are stretched to a point less than to the extension at maximum loading in order to maintain some of their strength in the spunbond layers.

The grammages of the layers described above concerns the finished material after stretching. Before stretching, the grammages for the individual layers were: inner film 40 g/m², outer spun bond layer 25 g/m², and adhesive layer 3 g/m². Since it is difficult to measure the grammages for individual layers after lamination and stretching, an approximation has been made on the basis of the grammages of the layers before lamination and stretching. The total grammage for the laminate before stretching was 93 g/m² and after stretching the grammage was 85 g/m², which corresponds to a deformation of about 10% it has been assumed that the deformation of the individual fiber layers and the film is identical, i.e. about 10%.

The reinforcing nonwoven strips were made of spunbond with a grammage of 20 g/m² in Table 1 and 20 g/m² or 30 g/m². As will be seen from Table 1, the tensile strength for the reinforced weld was 10 N/25.4 mm² greater than the weld consisting of only two elastic laminates. As will be seen from Table 2, gluing a reinforcing strip to the laminate at the back portion, which in this example is the portion that is disposed toward a stationary horn as the weld is formed using a transversely rotating anvil and a stationary horn, can provide further reinforcement.

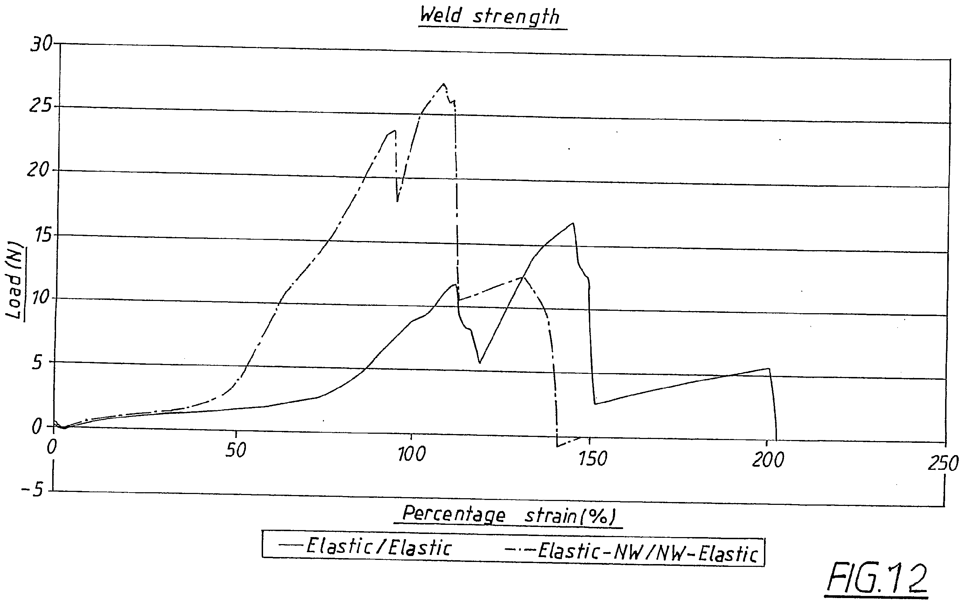

The particular values reflected in each table are of course due to the particular construction of the layers and the grammage. The welding method, bonder force setting, amplitude setting, and weld pattern also affect the strength. Therefore, Table 1 should not be directly compared with Table 2. However, the results shown in Table 1 illustrates clearly that a substantial reinforcement of the weld seam can be obtained with the aid of reinforcing nonwoven strips. The results shown in Table 2 show that further reinforcement of the weld seams can be obtained by adhesively affixing at least one reinforcing strip. Figure 12 shows the stress-strain diagram for the test that corresponds to the results in Table 1.

**TABLE 1. Tensile strength of welds formed between elastic laminates with and without reinforcing nonwoven strips.**

| **Elastic / Elastic (no reinforcement)** | | | **Elastic-NW-NW-Elastic (two reinforcing strips-no glue)** | | |
|---|---|---|---|---|---|
| Sample | Maximum Force (N) | Elongation at Max Load (%) | Sample | Maximum Force (N) | Elongation at Max Load (%) |
| 1 | 16.81 | 145 | 1 | 19.38 | 78 |
| 2 | 15.65 | 108 | 2 | 33.63 | 109 |
| 3 | 16.12 | 118 | 3 | 21.26 | 91 |
| 4 | 13.83 | 117 | 4 | 29.17 | 100 |
| 5 | 12.89 | 114 | 5 | 24.70 | 99 |
| 6 | 15.46 | 112 | 6 | 28.23 | 108 |
| 7 | 16.42 | 109 | 7 | 21.49 | 93 |
| 8 | 17.99 | 107 | 8 | 31.56 | 110 |
| 9 | 22.11 | 117 | 9 | 27.57 | 108 |
| 10 | 17.96 | 110 | 10 | 30.58 | 108 |
| **AVG** | 16.52 | 116 | **AVG** | 26.76 | 100 |
| **SD** | 2.54 | 11 | **SD** | 4.83 | 10 |
| **MIN** | 12.89 | 107 | **MIN** | 19.38 | 78 |
| **MAX** | 22.11 | 145 | **MAX** | 33.63 | 110 |

**TABLE 2. Tensile strength of reinforced welds formed between elastic laminates with and without adhesively affixing a reinforcing nonwoven strip to an elastic laminate layer.**

| | **No Glue** | | | **Strip Glued on Back Portion** | | |
|---|---|---|---|---|---|---|
| | 1 strip 20 g/m² | 2 strips 20 g/m² | 2 strips 30 g/m² | 20 g/m² | 20 g/m² + 20 g/m² on front without glue | 30 g/m² +30 g/m² on front without glue |
| AVG Right | 12.95 | 11.27 | 11.00 | 18.50 | 21.40 | 24.60 |
| SD Right | 1.50 | 1.05 | 1.31 | 1.90 | 3.00 | 3.10 |
| MIN Right | 10.00 | 8.50 | 8.70 | 14.80 | 16.00 | 18.30 |
| AVG Left | 13.33 | 12.56 | 12.70 | 16.50 | 22.80 | 23.70 |
| SD Left | 2.57 | 1.66 | 1.70 | 1.90 | 2.50 | 2.90 |
| MIN Left | 8.70 | 9.5 | 10.50 | 12.20 | 18.50 | 18.10 |

Practical tests have shown that the tensile strength of the weld seams in a direction transverse to the weld seam should be at least 5 N/25.4 mm, preferably 7 N/25.4 mm or more preferably at least 9 N/25.4 mm to ensure that diaper pants of reliable function are obtained. Across samples of 25 or more hygiene pants, minimum side seam strengths of at least 10 N/25.4 mm are preferred, or of at least 12 N/25.4 mm are most preferred, while average tensile strengths of at least 15 N/25.4 mm are preferred.

Hygiene pants when used should be able to be easily opened at the weld seams in order to make it easier to take them off. To obtain a reliable function in this respect, it is desirable that the breaking strength of the cover in the transverse direction exceeds the tensile strength for the reinforced weld seams so that the hygiene pants, when being taken off, do not fall apart in an uncontrolled manner and instead break at the seams. The fact that the reinforcements of the seams can be varied through the choice of nonwoven, the number of layers and the grammage of the reinforcement strips opens up a new possibility of varying the strength of the weld seam. This strength can also be varied through the choice of weld type and weld pattern.

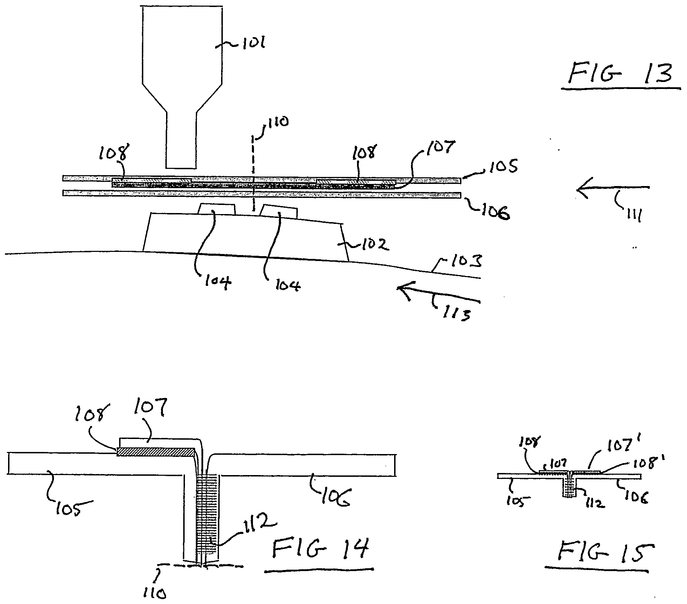

Figures 13-15 illustrate a method of making weld seam which does not form part of the invention. Said method makes a reinforced ultrasonic or thermally bonded weld seam where reinforcing material is adhesively attached to elastic laminate cover material proximate to the location of a weld between two layers of elastic laminate material. In Figure 13, elastic laminate material 105, 106 move transversely in direction 111 under a stationary ultrasonic horn 101. Anvil 102 bearing weld patterns 104 is attached to the surface of roller 103 moving in direction 113. Reinforcing nonwoven material 107 is attached the inner surface of elastic laminate 105, which is disposed towards the stationary horn, by adhesive glue 108. Glue 108 could extend the full length of reinforcing material 107, but it is preferable that glue 108 is positioned proximate to, but not at, the location where the weld will be formed. After welds are formed by compression of the laminates between horn 101 and weld patterns 104, the welded material is cut at line 110 to form a seam 112 as illustrated in FIG 14. In practical experience, the weld 112 tends to be stronger on the anvil side. By use of this method, any weakness in the weld 112 on the horn side is reinforced by the use of glue 108 to affix reinforcing material 107 to the horn-side elastic laminate 105. FIG 15 illustrates a seam formed by including an additional nonwoven reinforcing material 107'on the anvil side elastic laminate 106, which can be applied with or without glue 108'.

The hygiene pants described herein are not limited to the illustrative embodiments described above. Rather, a number of modifications are possible within the scope of the attached patent claims.

An elasticized cover does not need to be made of elastic laminate across the whole front portion and whole rear portion, and instead parts of the front portion and/or rear portion can be made of inelastic material. It is preferred that the hygiene pants are sufficiently elastic so that the pants can be put on and taken off with ease. For example, it may suffice for the rear portion to be made of elastic laminate and for the rest of the hygiene pants to be non-elastic.

Alternatively, the hygiene pants as described herein can have covers in the form of elastic laminates only in the side portions of the front portion and rear portion, and otherwise have central non-elastic parts along the front portion, rear portion and crotch portion cover in the transverse direction exceeds the tensile strength for the reinforced weld seams.

## Claims

1. Disposable hygiene pants with a longitudinal direction and a transverse direction, comprising in the longitudinal direction, a front portion (2), an intermediate crotch portion (4) provided with two leg cut outs, and a rear portion (3), the outer longitudinal edge portions (21, 22) of the front portion being connected by longitudinal weld seams (7, 8) to the outer longitudinal edge portions (31, 32) of the rear portion (3) thereby forming a waist opening (9) that is delimited by the outer transverse edge portions (22, 33) of the front portion and of the rear portion, and forming two leg openings (5, 6) that are outwardly delimited in the transverse direction by the inner end portions of the weld seams, wherein the hygiene pants comprise an outer elastic cover (10) comprising an elastic laminate comprising an elastic film applied to a nonwoven material and an absorption unit (11) that extends in the longitudinal direction across at least part of the crotch portion (4), wherein the hygiene pants are **characterized in that** said weld seams (7, 8) are each reinforced by one or more pieces of reinforcing material (15, 16, 17, 18, 19, 20, 35) containing thermoplastic fibers, and wherein said reinforcing material has been affixed by adhesive (25, 26, 27, 28, 29, 30, 37) to inner surfaces of the front portion (21, 22) and/or the rear portion of the elastic outer cover (10) proximate to the weld seams (7,8) such that the reinforcing material extends into said weld seams (7, 8), and **in that** the tensile strength of the weld seams, in a direction transverse to the weld seam and in said transverse direction, exceeds 5 N/25.4 mm at least in the portions reinforced with said reinforcing material (15, 16, 17, 18, 19, 20, 35), and **in that** the breaking strength of said cover in the transverse direction exceeds the tensile strength for the reinforced weld seams.

2. Hygiene pants according to Claim 1, wherein the cover comprises an elastic laminate comprising an elastic film applied between two nonwoven layers.

3. The hygiene pants according to Claim 1 or 2, wherein the reinforcing material is affixed by glue that is applied proximate to, but not extending into, the area of the weld seam.

4. Hygiene pants according to any one of Claims 1 to 3, wherein said reinforcing material is a nonwoven strip.

5. Hygiene pants according to any one of Claims 1 to 4, **characterized in that** said reinforcing material (15, 16, 18, 35) is adhesively affixed at least to the longitudinal edge portions of the rear portion of the cover.

6. Hygiene pants according to any one of Claims 1 to 4, **characterized in that** said reinforcing material (15, 16, 35) extends over the whole longitudinal length of the longitudinal edge portions.

7. Hygiene pants according to any one of Claims 1 to 4, **characterized in that** each weld seam (7, 8) is reinforced by a single piece of reinforcing material (15, 16, 17, 18, 19, 20, 35).

8. Hygiene pants according to any one of Claims 1 to 4, **characterized in that** reinforcing material is adhesively affixed to the cover proximate to the weld seams in each of said longitudinal edge portions of either the front portion or the rear portion and that reinforcing material is applied with or without adhesive to each longitudinal edge portion of the other of the front portion or the rear portion, as a result of which the weld seams, at least in their reinforced areas, comprise two layers of said reinforcing nonwoven strips.

9. Hygiene pants according to any one of Claims 1 to 4, **characterized in that** said reinforcing material is a single piece of reinforcing material extending transversely across the hygiene pants.

10. Hygiene pants according to Claim 9, **characterized in that**, in the longitudinal direction of the hygiene pants, said single piece of reinforcing material extends only across the inner end portions of the two edge portions.

11. Hygiene pants according to Claim 9, **characterized in that**, in the longitudinal direction of the hygiene pants, said single piece of reinforcing material extends across substantially all of the two edge portions.

12. Hygiene pants according to any of the preceding claims, **characterized in that** said tensile strength across the weld seams (7, 8) in the reinforced portions exceeds 10 N/25.4 mm.

13. Hygiene pants according to any of the preceding claims, **characterized in that** said tensile strength across the weld seams (7, 8) in the reinforced portions exceeds 12 N/25.4 mm.

14. Hygiene pants according to any of the preceding claims, **characterized in that** said tensile strength across the weld seams (7, 8) in the reinforced portions exceeds 15 N/25.4 mm.

15. Hygiene pants according to any of the preceding claims, **characterized in that** said tensile strength across the weld seams (7, 8) in the reinforced portions exceeds 20 N/25.4 mm.

16. Hygiene pants according to any of the preceding claims, **characterized in that** when the weld seam is made, either the front or the back portion is disposed toward an ultrasonic horn and the other of the front or back portion is disposed toward an anvil and wherein pieces of reinforcing material are affixed to the inner surface of the portion that is contacted by the ultrasonic horn.

17. Hygiene pants according to Claim 16, wherein pieces of reinforcing material are affixed only to the inner surface of the portion that is contacted by the ultrasonic horn

18. Hygiene pants according to Claim 16 or Claim 17, wherein when the weld seam is being made, the ultrasonic horn is stationary with respect to the transverse direction of the pant and the anvil and pant are moving in the transverse direction of the pant.

19. Hygiene pants according to any of Claims 1 to 15, **characterized in that** the strength of the bond in said weld seams between either the front or the back portion and the reinforcing material is weaker than between the other of the front or the back portion and wherein the reinforcing material is adhesively affixed to the inner surface of whichever of the front or back portion is on the weaker side.

## Patentansprüche

1. Einweghygienehose mit einer Längsrichtung und einer Querrichtung, in der Längsrichtung aufweisend einen Vorderabschnitt (2), einen mittleren Schrittabschnitt (4), der mit zwei Beinausschnitten versehen ist, und einen Rückabschnitt (3), wobei die äußeren Längsrandabschnitte (21, 22) des Vorderabschnitts durch Längsschweißnähte (7, 8) mit den äußeren Längsrandabschnitten (31, 32) des Rückabschnitts (3) verbunden sind, wodurch sie eine Hüftöffnung (9) ausbilden, die durch die äußeren Querrandabschnitte (22, 33) des Vorderabschnitts und des Rückabschnitts begrenzt sind, und zwei Beinöffnungen (5, 6) ausbilden, die nach außen in der Querrichtung durch die inneren Endabschnitte der Schweißnähte begrenzt sind, wobei die Hygienehose eine äußere elastische Abdeckung (10) aufweist, die ein elastisches Schichtelement aufweist, das einen elastischen Film, der auf ein Vliessmaterial aufgebracht ist, und eine Absorptionseinheit (11) aufweist, die sich in der Längsrichtung über mindestens einen Teil des Schrittabschnitts (4) erstreckt, wobei die Hygienehose **dadurch gekennzeichnet ist, dass** die Schweißnähte (7, 8) jeweils durch ein oder mehrere Verstärkungsmaterialstücke (15, 16, 17, 18, 19, 20, 35) verstärkt sind, die thermoplastische Fasern aufweisen, und wobei das Verstärkungsmaterial so durch ein Haftmittel (25, 26, 27, 28, 29, 30, 37) an inneren Oberflächen des Vorderabschnitts (21, 22) und/oder des Rückabschnitts der elastischen äußeren Abdeckung (10) in der Nähe zu den Schweißnähten (7, 8) angebracht ist, dass sich das Verstärkungsmaterial in die Schweißnähte (7, 8) erstreckt, und wobei die Zugfestigkeit der Schweißnähte in einer Richtung quer zur Schweißnaht und in der Querrichtung 5 N/24,4 mm zumindest in den Abschnitten übersteigt, die mit dem Verstärkungsmaterial (15, 16, 17, 18, 19, 20, 35) verstärkt sind, und wobei die Bruchfestigkeit der Abdeckung in der Querrichtung die Zugfestigkeit der verstärkten Schweißnähte übersteigt.

2. Hygienehose nach Anspruch 1, wobei die Abdeckung ein elastisches Schichtelement aufweist, das einen elastischen Film aufweist, der zwischen den Vliessschichten aufgebracht ist.

3. Hygienehose nach Anspruch 1 oder 2, wobei das Verstärkungsmaterial durch einen Kleber angebracht ist, der in der Nähe des Gebiets der Schweißnaht aufgebracht ist, sich jedoch nicht hinein erstreckt.

4. Hygienehose nach einem der Ansprüche 1 bis 3, wobei das Verstärkungsmaterial ein Vliessstreifen ist.

5. Hygienehose nach einem der Ansprüche 1 bis 4, **dadurch gekennzeichnet, dass** das Verstärkungsmaterial (15, 16, 18, 35) haftend zumindest an den Längsrandabschnitten des Rückabschnitts der Abdeckung angebracht ist.

6. Hygienehose nach einem der Ansprüche 1 bis 4, **dadurch gekennzeichnet, dass** sich das Verstärkungsmaterial (15, 16, 35) über die gesamte Längslänge der Längsrandabschnitte erstreckt.

7. Hygienehose nach einem der Ansprüche 1 bis 4, **dadurch gekennzeichnet, dass** jede Schweißnaht (7, 8) durch ein einzelnes Verstärkungsmaterialstück (15, 16, 17, 18, 19, 20, 35) verstärkt ist.

8. Hygienehose nach einem der Ansprüche 1 bis 4, **dadurch gekennzeichnet, dass** das Verstärkungsmaterial in der Nähe der Schweißnaht in jedem der Längsrandabschnitte von entweder dem Vorderabschnitt oder dem Rückabschnitt haftend an der Abdeckung angebracht ist, und dass Verstärkungsmaterial mit oder ohne ein Haftmittel auf jeden Längsrandabschntit des anderen des Vorderabschnitts oder des Rückabschnitts aufgebracht ist, wodurch die Schweißnähte, zumindest in ihren verstärkten Gebieten, zwei Schichten der verstärkenden Vliessstreifen aufweisen.

9. Hygienehose nach einem der Ansprüche 1 bis 4, **dadurch gekennzeichnet, dass** das Verstärkungsmaterial ein einzelnes Verstärkungsmaterialstück ist, das sich quer über die Hygienehose erstreckt.

10. Hygienehose nach Anspruch 9, **dadurch gekennzeichnet, dass** sich das einzelne Verstärkungsmaterialstück in der Längsrichtung der Hygienehose nur über die inneren Endabschnitte der zwei Randabschnitte erstreckt.

11. Hygienehose nach Anspruch 9, **dadurch gekennzeichnet, dass** sich das einzelne Verstärkungsmaterialstück in der Längsrichtung der Hygienehose im Wesentlichen über die Gesamtheit der zwei Randabschnitte erstreckt.

12. Hygienehose nach einem der vorangegangenen Ansprüche, **dadurch gekennzeichnet, dass** die Zugfestigkeit über die Schweißnähte (7, 8) in den verstärkten Abschnitten 10 N/25,4 mm übersteigt.

13. Hygienehose nach einem der vorangegangenen Ansprüche, **dadurch gekennzeichnet, dass** die Zugfestigkeit über die Schweißnähte (7, 8) in den verstärkten Abschnitten 12 N/25,4 mm übersteigt.

14. Hygienehose nach einem der vorangegangenen Ansprüche, **dadurch gekennzeichnet, dass** die Zugfestigkeit über die Schweißnähte (7, 8) in den verstärkten Abschnitten 15 N/25,4 mm übersteigt.

15. Hygienehose nach einem der vorangegangenen Ansprüche, **dadurch gekennzeichnet, dass** die Zugfestigkeit über die Schweißnähte (7, 8) in den verstärkten Abschnitten 20 N/25,4 mm übersteigt.

16. Hygienehose nach einem der vorangegangenen Ansprüche, **dadurch gekennzeichnet, dass**, wenn die Schweißnaht hergestellt wird, entweder der Vorder- oder der Rückabschnitt in Richtung eines Ultraschallhorns angeordnet ist und der Andere des Vorder- oder des Rückabschnitts in Richtung eines Ambosses angeordnet ist, und wobei Verstärkungsmaterialstücke an der inneren Oberfläche des Abschnitts angebracht werden, der mit Ultraschallhorn in Verbindung steht.

17. Hygienehose nach Anspruch 16, wobei Verstärkungsmaterialstücke nur an der inneren Oberfläche des Abschnitts angebracht werden, der mit dem Ultraschallhorn in Verbindung steht.

18. Hygienehose nach Anspruch 16 oder 17, wobei, wenn die Schweißnaht hergestellt wird, das Ultraschallhorn in Bezug auf die Querrichtung der Hose stationär ist und sich der Amboss und die Hose in der Querrichtung der Hose bewegen.

19. Hygienehose nach einem der Ansprüche 1 bis 15, **dadurch gekennzeichnet, dass** die Stärke der Bindung in den Schweißnähten entweder zwischen dem Vorder- oder dem Rückabschnitt und dem Verstärkungsmaterial schwächer ist als zwischen dem anderen des Vorder- oder des Rückabschnitts, und wobei das Verstärkungsmaterial haftend mit der inneren Oberfläche von dem Vorder- oder Rückabschnitt verbunden ist, der sich auf der schwächeren Seite befindet.

## Revendications

1. Culottes hygiéniques jetables avec une direction longitudinale et une direction transversale, comprenant dans la direction longitudinale une partie avant (2), une partie d'entrejambe intermédiaire (4) dotée de deux découpes de jambes, et une partie arrière (3), les parties de bord longitudinales externes (21, 22) de la partie avant étant reliées par des soudures longitudinales (7, 8) aux parties de bord longitudinales externes (31, 32) de la partie arrière (3), formant ainsi une ouverture de taille (9) qui est délimitée par les parties de bord transversales externes (22, 33) de la partie avant et de la partie arrière, et formant deux ouvertures de jambes (5, 6) qui sont délimitées à l'extérieur dans la direction transversale par les parties d'extrémité internes des soudures, dans lesquelles les culottes hygiéniques comprennent une gaine élastique externe (10) comprenant un lamellé élastique comprenant un film élastique appliqué sur un matériau non tissé et une unité d'absorption (11) qui s'étend dans la direction longitudinale à travers au moins une portion de la partie d'entrejambe (4), dans lesquelles les culottes hygiéniques sont **caractérisées en ce que** lesdites soudures (7, 8) sont chacune renforcées par une ou plusieurs pièces de matériau de renforcement (15, 16, 17, 18, 19, 20, 35) contenant des fibres thermoplastiques, et dans lesquelles ledit matériau de renforcement a été fixé par adhésif (25, 26, 27, 28, 29, 30, 37) à des surfaces internes de la partie avant (21, 22) et/ou de la partie arrière de la gaine externe élastique (10) à proximité des soudures (7, 8) de telle sorte que le matériau de renforcement s'étend dans lesdites soudures (7, 8), et **en ce que** la résistance à la traction des soudures, dans une direction transversale à la soudure et dans ladite direction transversale, dépasse 5 N/25,4 mm au moins dans les parties renforcées avec ledit matériau de renforcement (15, 16, 17, 18, 19, 20, 35), et **en ce que** la résistance à la rupture de ladite gaine dans la direction transversale dépasse la résistance à la traction pour les soudures renforcées.

2. Culottes hygiéniques selon la revendication 1, dans lesquelles la gaine comprend un lamellé élastique comprenant un film élastique appliqué entre deux couches non tissées.

3. Culottes hygiéniques selon la revendication 1 ou 2, dans lesquelles le matériau de renforcement est fixé par une colle qui est appliquée à proximité de, mais ne s'étendant pas dans, la zone de la soudure.

4. Culottes hygiéniques selon l'une quelconque des revendications 1 à 3, dans lesquelles ledit matériau de renforcement est une bande non tissée.

5. Culottes hygiéniques selon l'une quelconque des revendications 1 à 4, **caractérisées en ce que** ledit matériau de renforcement (15, 16, 18, 35) et fixé de manière adhésive au moins aux parties de bord longitudinales de la partie arrière de la gaine.

6. Culottes hygiéniques selon l'une quelconque des revendications 1 à 4, **caractérisées en ce que** ledit matériau de renforcement (15, 16, 35) s'étend sur la totalité de la longueur longitudinale des parties de bord longitudinales.

7. Culottes hygiéniques selon l'une quelconque des revendications 1 à 4, **caractérisées en ce que** chaque soudure (7, 8) est renforcée par une pièce unique de matériau de renforcement (15, 16, 17, 18, 19, 20, 35).

8. Culottes hygiéniques selon l'une quelconque des revendications 1 à 4, **caractérisées en ce que** le matériau de renforcement est fixé de manière adhésive à la gaine à proximité des soudures dans chacune desdites parties de bord longitudinales soit de la partie avant soit de la partie arrière et **en ce que** le matériau de renforcement est appliqué avec ou sans adhésif à chaque partie de bord longitudinale de l'autre de la partie avant ou de la partie arrière, suite à quoi les soudures, au moins dans leurs zones renforcées, comprennent deux couches desdites bandes non tissées de renforcement.

9. Culottes hygiéniques selon l'une quelconque des revendications 1 à 4, **caractérisées en ce que** ledit matériau de renforcement est une pièce unique de matériau de renforcement s'étend de manière transversale à travers les culottes hygiéniques.

10. Culottes hygiéniques selon la revendication 9, **caractérisées en ce que**, dans la direction longitudinale des culottes hygiéniques, ladite pièce unique de matériau de renforcement s'étend uniquement à travers les parties d'extrémité internes des deux parties de bord.

11. Culottes hygiéniques selon la revendication 9, **caractérisées en ce que**, dans la direction longitudinale des culottes hygiéniques, ladite pièce unique de matériau de renforcement s'étend à travers sensiblement la totalité des deux parties de bord.

12. Culottes hygiéniques selon l'une quelconque des revendications précédentes, **caractérisées en ce que** ladite résistance à la traction à travers les soudures (7, 8) dans les parties renforcées dépasse 10 N/25,4 mm.

13. Culottes hygiéniques selon l'une quelconque des revendications précédentes, **caractérisées en ce que** ladite résistance à la traction à travers les soudures (7, 8) dans les parties renforcées dépasse 12 N/25,4 mm.

14. Culottes hygiéniques selon l'une quelconque des revendications précédentes, **caractérisées en ce que** ladite résistance à la traction à travers les soudures (7, 8) dans les parties renforcées dépasse 15 N/25,4 mm.

15. Culottes hygiéniques selon l'une quelconque des revendications précédentes, **caractérisées en ce que** ladite résistance à la traction à travers les soudures (7, 8) dans les parties renforcées dépasse 20 N/25,4 mm.

16. Culottes hygiéniques selon l'une quelconque des revendications précédentes, **caractérisées en ce que** lorsque la soudure est réalisée, soit la partie avant soit la partie arrière est disposée vers une sonotrode et l'autre de la partie avant ou arrière est disposée vers une enclume et dans lesquelles des pièces de matériau de renforcement sont fixées à la surface interne de la partie qui entre en contact avec la sonotrode.

17. Culottes hygiéniques selon la revendication 16, dans lesquelles des pièces de matériau de renforcement sont fixées uniquement à la surface interne de la partie qui entre en contact avec la sonotrode.

18. Culottes hygiéniques selon la revendication 16 ou la revendication 17, dans lesquelles lorsque la soudure est réalisée, la sonotrode est stationnaire par rapport à la direction transversale de la culotte et l'enclume et la culotte se déplacent dans la direction transversale de la culotte.

19. Culottes hygiéniques selon l'une quelconque des revendications 1 à 15, **caractérisées en ce que** la résistance de la liaison dans lesdites soudures entre soit la partie avant soit la partie arrière et le matériau de renforcement est plus faible qu'entre l'autre de la partie avant ou la partie arrière et dans lesquelles le matériau de renforcement est fixé de manière adhésive à la surface interne de la partie avant ou arrière qui est sur le côté plus faible.
